# EUROPEAN PATENT APPLICATION

(11) **EP 2 172 216 A2**
(43) Date of publication of application: **07.04.2010**
(21) Application number: 09166906.9
(22) Date of filing: 10.03.1998
(51) Int. Cl.: A61K 39/39

(54) **Use of nucleic acids containing unmethylated CpG dinucleotide as an adjuvant**

(30) Priority: 10.03.1997 US 40376 P
(62) Divisional of application: 98912919.2
(71) Applicant: Ottawa Hospital Research Institute, Ottawa, ON K1Y 4E9 (CA); Coley Pharmaceutical GmbH, 40225 Düsseldorf (DE); University of Iowa Research Foundation, Iowa City, IA 52242-5500 (US); The United States of America as represented by the Secretary Department of Health and Human Services, Washington, DC (US)
(72) Inventor: Davis, Heather, Dunrobin, Ontario K0A 1T0 (CA); Krieg, Arthur, Wellesley, MA 02482 (US); Schorr, Joachim, 40724 Hilden (DE); Klinman, Dennis, Potomac, MD 20854 (US)
(74) Representative: Jump, Timothy John Simon

(57) **Abstract**

The present invention is based on the finding that nucleic acids containing at least one unmethylated cytosine-guanine (CpG) dinucleotide affect immune responses in a subject. These nucleic acids containing at least one unmethylated cytosine-guanine (CpG) dinucleotide can be used to induce an immune response in a subject. The method includes administering to the subject a therapeutically effective amount of nucleic acid encoding an antigenic polypeptide, and a therapeutically effective amount of an oligonucleotide containing at least one unmethylated CpG dinucleotide. The invention also provides a method for treating a subject having or at risk of having viral-mediated disorder, comprising administering to the subject a therapeutically effective amount of a nucleic acid encoding an antigenic polypeptide and an effective amount of an oligonucleotide containing at least one unmethylated CpG dinucleotide

## Description

### FIELD OF THE INVENTION

This invention relates to generally to adjuvants, and specifically to the use of oligonucleotides having at least one unmethylated CpG dinucleotide (CpG ODN) as an adjuvant.

### BACKGROUND OF THE INVENTION

Bacterial DNA, but not vertebrate DNA, has direct immunostimulatory effects on peripheral blood mononuclear cells (PBMC) *in vitro* (Krieg *et al*., 1995). This lymphocyte activation is due to unmethylated CpG dinucleotides, which are present at the expected frequency in bacterial DNA (1/16), but are under-represented (CpG suppression, 1/50 to 1/60) and methylated in vertebrate DNA. Activation may also be triggered by addition of synthetic oligodeoxynucleotides (ODN) that contain an unmethylated CpG dinucleotide in a particular sequence context. It appears likely that the rapid immune activation in response to CpG DNA may have evolved as one component of the innate immune defense mechanisms that recognize structural patterns specific to microbial molecules.

CpG DNA induces proliferation of almost all (>95%) B cells and increases immunoglobulin (Ig) secretion. This B cell activation by CpG DNA is T cell independent and antigen non-specific. However, B cell activation by low concentrations of CpG DNA has strong synergy with signals delivered through the B cell antigen receptor for both B cell proliferation and Ig secretion (Krieg *et al.,* 1995). This strong synergy between the B cell signaling pathways triggered through the B cell antigen receptor and by CpG DNA promotes antigen specific immune responses. In addition to its direct effects on B cells, CpG DNA also directly activates monocytes, macrophages, and dendritic cells to secrete a variety of cytokines, including high levels of IL-12 (Klinman *et al.,* 1996; Halpern *et al*., 1996; Cowdery *et al.,* 1996). These cytokines stimulate natural killer (NK) cells to secrete g-interferon (IFN-g) and have increased lytic activity (Klinman *et al.,* 1996, *supra*; Cowdery *et al.,* 1996, *supra*; Yamamoto *et al*., 1992; Ballas *et al*., 1996). Overall, CpG DNA induces a Th1 like pattern of cytokine production dominated by IL-12 and IFN-g with little secretion of Th2 cytokines (Klinman *et al*., 1996). The strong direct effects (T cell independent) of CpG DNA on B cells, as well as the induction of cytokines which could have indirect effects on B-cells via T-help pathways, suggests utility of CpG DNA in the form of ODN as a vaccine adjuvant.

A DNA vaccine induces immune responses against an antigenic protein expressed *in vivo* from an introduced gene. The DNA vaccine is most often in the form of a plasmid DNA expression vector produced in bacteria and then purified and delivered to muscle or skin (see Vogel and Sarver, 1995; Brazolot Millan and Davis, 1997; Donnelly *et al.,* 1996). DNA vaccines have been demonstrated to show efficacy against numerous viral, bacterial and parasitic diseases in animal models. Almost all studies show induction of very strong and long-lasting humoral and cell-mediated immune responses, and protection against live pathogen challenge (where it could be evaluated). The efficacy of DNA vaccines is attributed, at least in part, to the continuous *in vivo* synthesis of antigen that leads to efficient antigen presentation. In particular, endogenously-synthesized antigen is presented by class I MHC, leading to induction of CD8+ cytotoxic T lymphocytes (CTL). In contrast, most whole killed and subunit vaccines, where antigen is processed solely in the exogenous form, often fail to induce CTL. More recently however, it has been shown that the presence of unmethylated CpG motifs in the DNA vaccines is essential for the induction of immune responses against the antigen (Sato *et al.,* 1996).

Hepatitis B virus (HBV) poses a serious world-wide health problem. The current HBV vaccines are subunit vaccines containing particles of HBV envelope protein(s) which include several B and T cell epitopes known collectively as HBV surface antigen (HBsAg). The HBsAg particles may be purified from the plasma of chronically infected individuals or more commonly are produced as recombinant proteins. These vaccines induce antibodies against HBsAg (anti-HBs), which confer protection if present in titers 10 milli-Intemational Units per milliliter (mIU/ml) (Ellis, 1993). While the subunit vaccines are safe and generally efficacious, they fail to meet all current vaccination needs. For example, early vaccination of infants born to chronically infected mothers, as well as others in endemic areas, drastically reduces the rate of infection, but a significant proportion of these babies will still become chronically infected themselves. This could possibly be reduced if high titers of anti-HBs antibodies could be induced earlier and if there were HBV-specific CTL. In addition, there are certain individuals who fail to respond (non-responders) or do not attain protective levels of immunity (hypo-responders). Finally, there is an urgent need for an effective treatment for the estimated 350 million chronic carriers of HBV and a therapeutic vaccine could meet this need.

### SUMMARY OF THE INVENTION

The present invention is based on the finding that nucleic acids containing at least one unmethylated cytosine-guanine (CpG) dinucleotide affect the immune response in a subject by activating natural killer cells (NK) or redirecting a subject's immune response from a Th2 to a Th1 response by inducing monocytic and other cells to produce Th1 cytokines. These nucleic acids containing at least one unmethylated CpG can be used as an adjuvant, specifically to induce an immune response against an antigenic protein.

In one embodiment, the invention provides a method of inducing an immune response in a subject by administering to the subject a therapeutically effective amount of a nucleic acid encoding an antigenic protein and a therapeutically effective amount of an oligonucleotide containing at least one unmethylated CpG dinucleotide.

In another embodiment, the invention provides a method for treating a subject having or at risk of having a virally mediated disorder by administering to the subject a therapeutically effective amount of a nucleic acid encoding an antigenic protein and an effective amount of an oligonucleotide containing at least one unmethylated CpG dinucleotide.

In further embodiment, the invention provides a method for treating a subject having or at risk of having a chronic viral infection by administering to the subject an effective amount of an antigenic polypeptide and an effective amount of an oligonucleotide containing at least one unmethylated CpG dinucleotide.

In another embodiment, a pharmaceutical composition containing an immumostimulatory CpG oligonucleotide and a nucleic acid encoding an antigenic protein in a pharmaceutically acceptable carrier is provided.

### BRIEF DESCRIPTION OF THE DRAWINGS

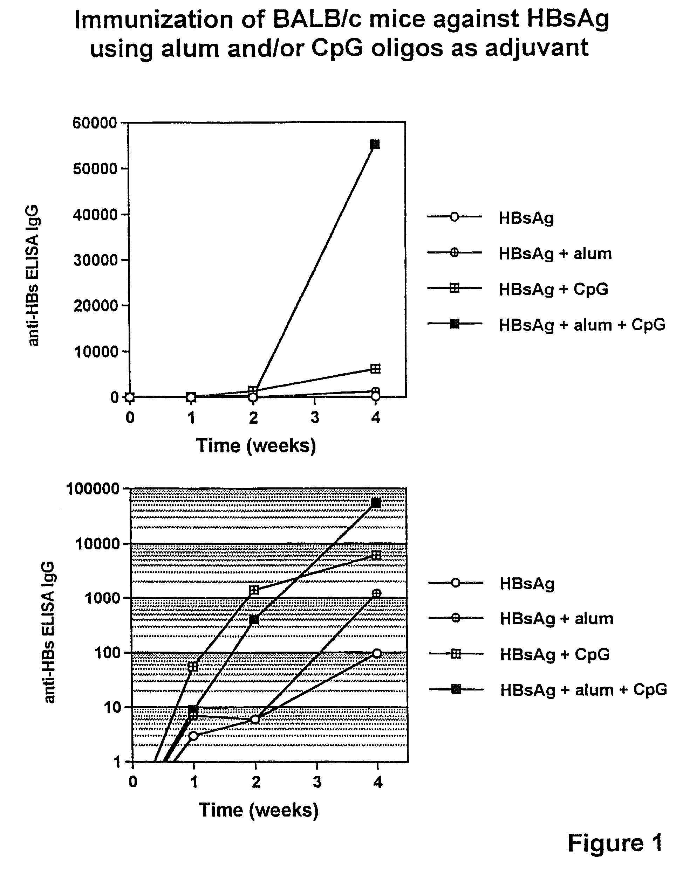

FIG. 1 is a graph illustrating humoral responses in BALB/c mice immunized with 1 g recombinant HBsAg protein alone, adsorbed onto alum (25 mg Al³⁺/mg HBsAg), with 100 g of immunostimulatory CpG ODN, or with both alum and CpG ODN. Each point represents the group mean (n=10) for titers of anti-HBs (total IgG) as determined in triplicate by end-point dilution ELISA assay. End-point titers were defined as the highest plasma dilution that resulted in an absorbance value (OD 450) two times greater than that of control non-immune plasma with a cut-off value of 0.05. The upper graph shows results on a linear scale and the lower graph shows results on a logarithmic scale (log₁₀).

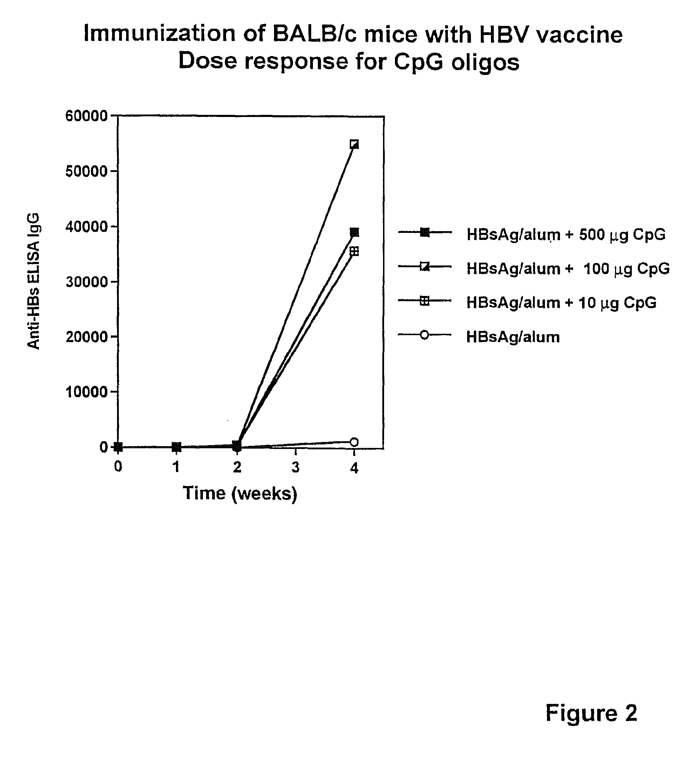

FIG. 2 is a graph illustrating humoral responses in BALB/c mice immunized with 1 g recombinant HBsAg protein with alum and with 0, 10, 100 or 500 g of CpG ODN added. Each point represents the group mean (n=10) for anti-HBs titers (total IgG) as determined by end-point dilution ELISA assay.

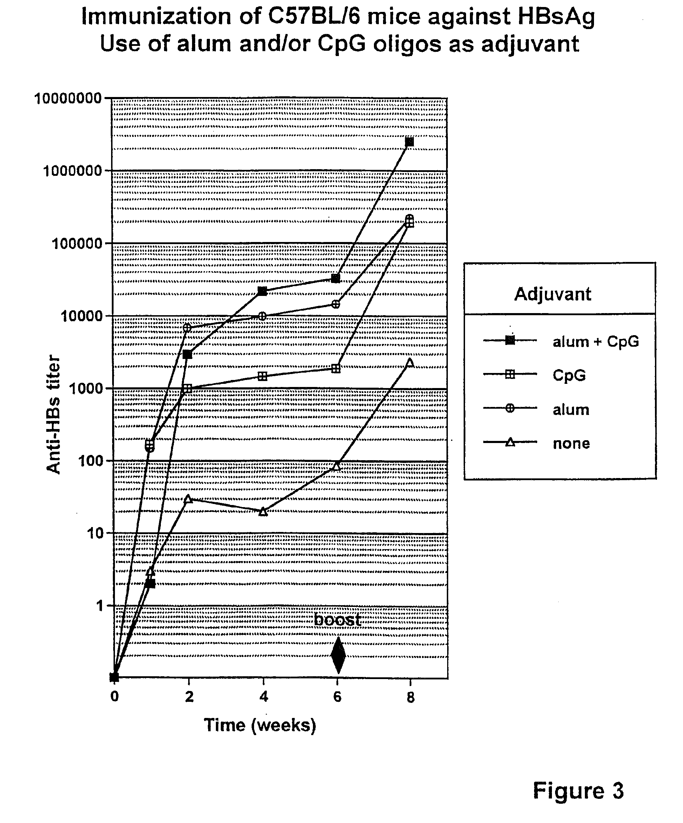

FIG. 3 is a graph of humoral responses in C57BL/6 mice immunized with 1 g recombinant HBsAg protein without adjuvant, with alum, with 100 g of CpG ODN, or with both alum and CpG ODN. Mice were boosted in the identical fashion after 6 weeks. Each point represents the group mean (n=5) for anti-HBs titers (total IgG) as determined by end-point dilution ELISA assay.

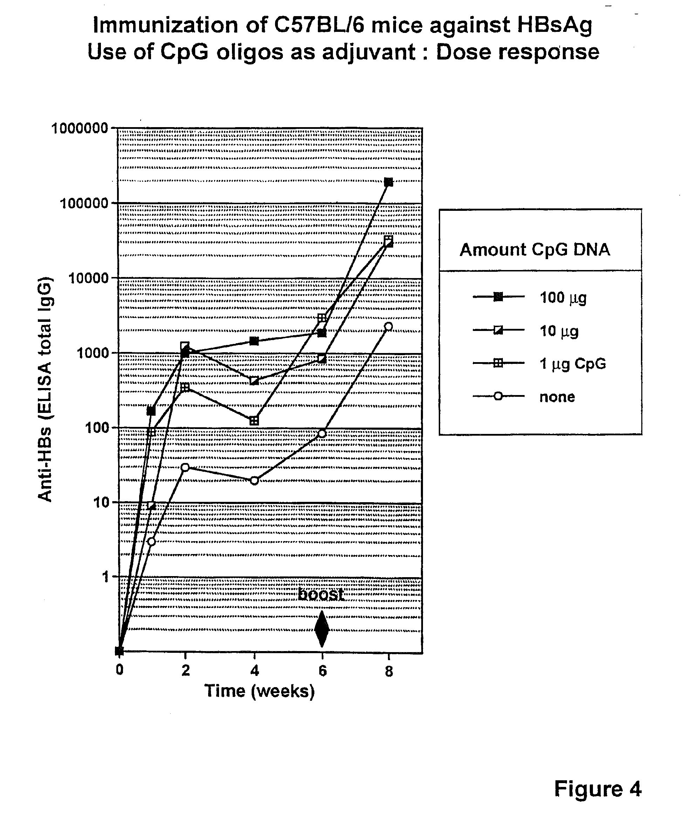

FIG. 4 is a graph of humoral responses in C57BL/6 mice immunized with 1 g recombinant HBsAg protein without adjuvant, or with 1, 10 or 100 g of CpG ODN. Mice were boosted in the identical fashion after 6 weeks. Each point represents the group mean (n=5) for anti-HBs titers (total IgG) as determined by end-point dilution ELISA assay.

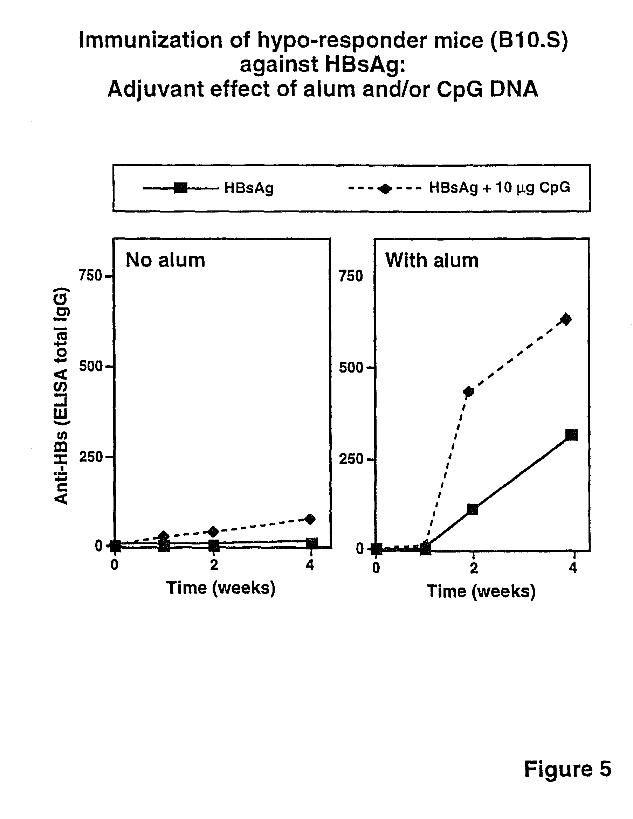

FIG. 5 is a graph of humoral responses in B10.S hypo-responder mice immunized with 1 g recombinant HBsAg protein without adjuvant, with alum, and/or with 10 g of CpG ODN. Each point represents the group mean (n=5) for anti-HBs titers (total IgG) as determined by end-point dilution ELISA assay.

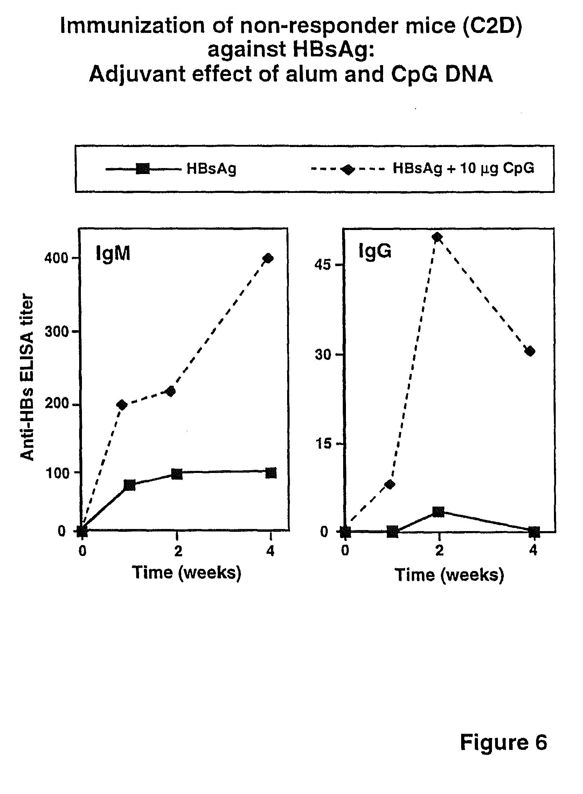

FIG. 6 is a graph of humoral responses in C2D non-responder mice immunized with 1 g recombinant HBsAg protein with alum or with alum plus 10 g of CpG ODN. Each point represents the group mean (n=5) for anti-HBs titers (total IgM or IgG) as determined by end-point dilution ELISA assay.

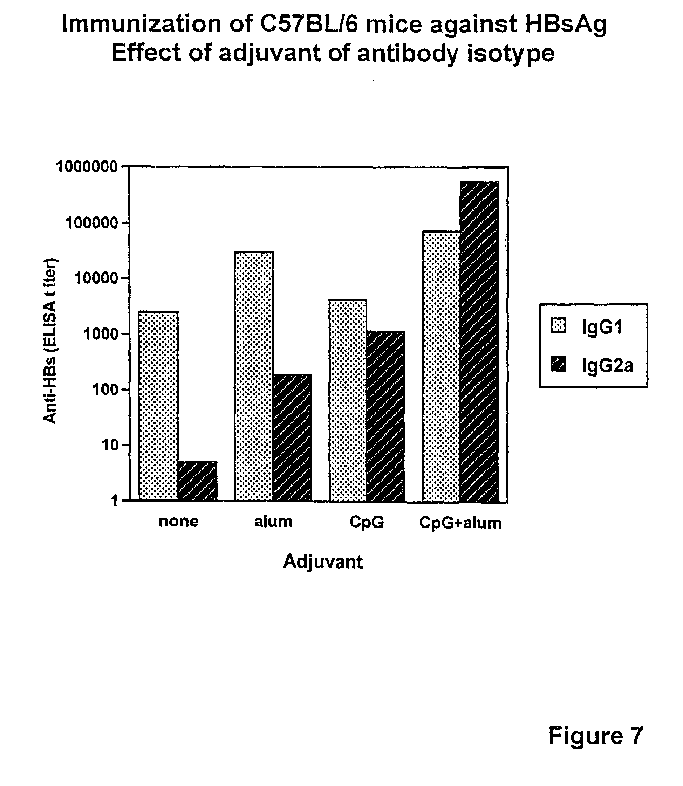

FIG. 7 is a bar graph illustrating humoral responses in C57BL/6 mice at 8 weeks after immunization with 1 g recombinant HBsAg protein without adjuvant, with alum, with 100 g of CpG ODN, or with both alum and CpG ODN. Mice had been boosted in the identical fashion at 6 weeks. Each point represents the group mean (n=5) for anti-HBs titers (IgG1 and IgG2a isotypes) as determined by end-point dilution ELISA assay.

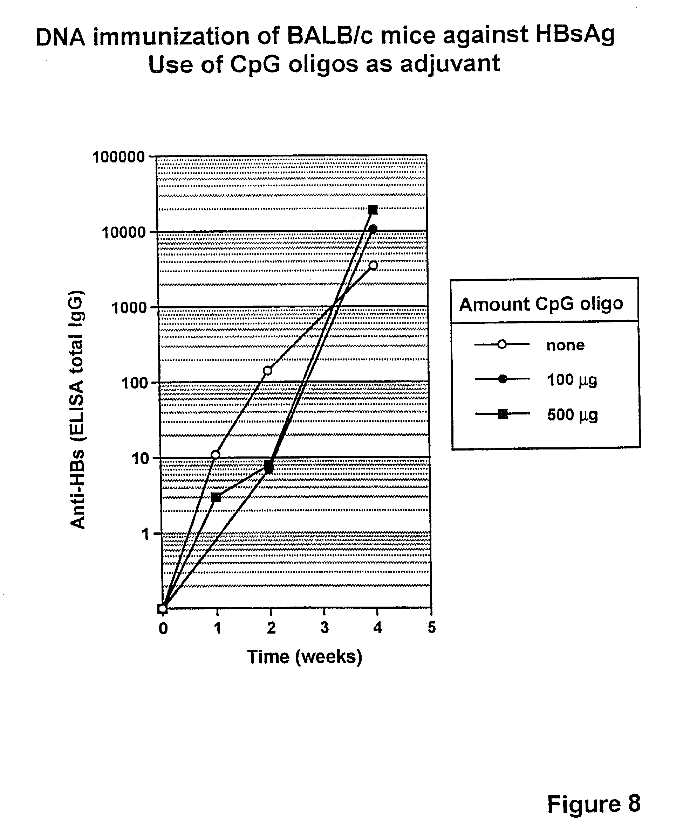

FIG. 8 is a graph of humoral responses in BALB/c mice immunized with 10 g HBsAg-expressing DNA vaccine (pCMV-S) injected alone or with 100 or 500 g of CpG ODN added. Each point represents the group mean (n=10) for anti-HBs titers (total IgG) as determined by end-point dilution ELISA assay.

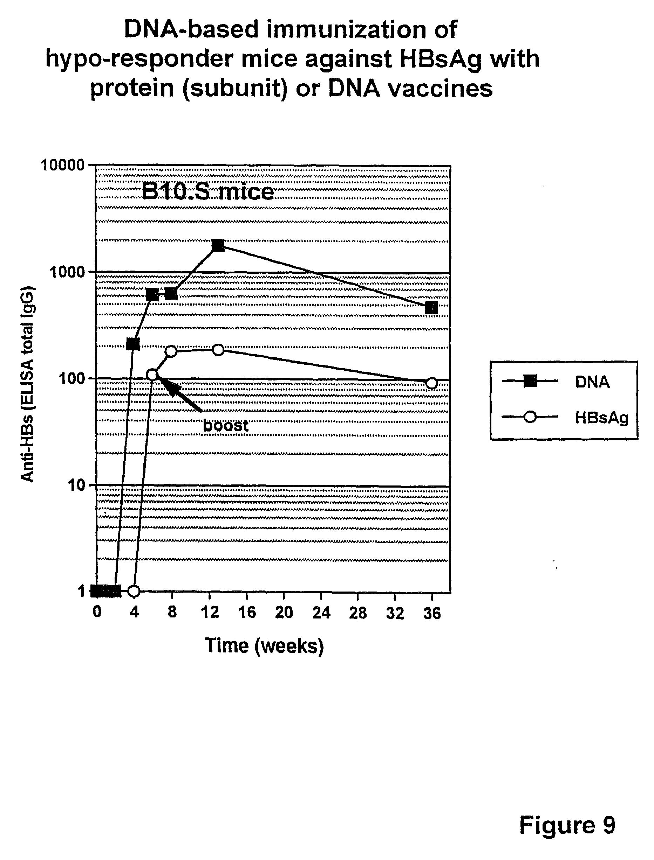

FIG. 9 is a graph of humoral responses in B10.S mice immunized with 2 g recombinant HBsAg protein without adjuvant or with 50 g pCMV-S DNA vaccine. Each point represents the group mean (n=5) for anti-HBs titers (total IgG) as determined by end-point dilution ELISA assay.

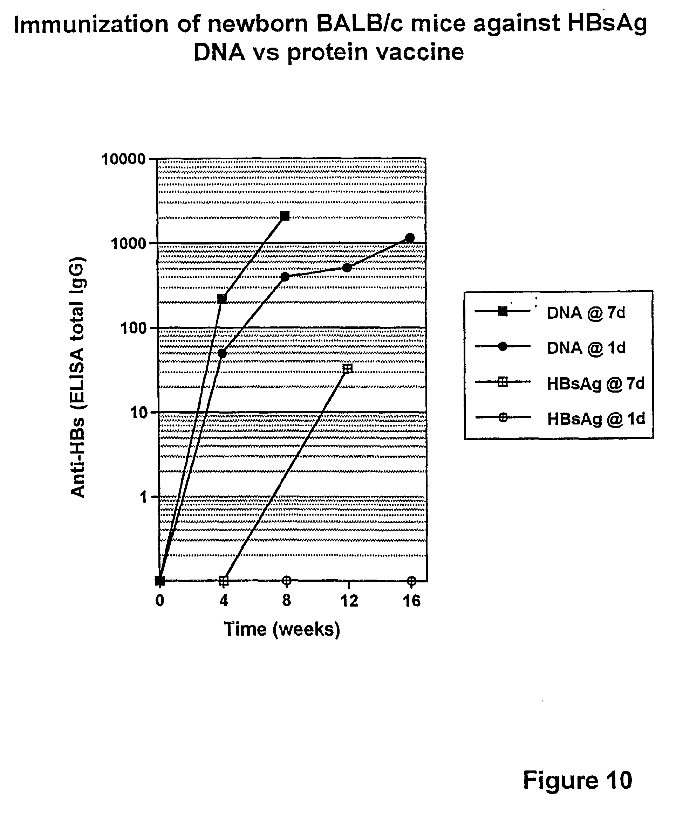

FIG. 10 is a graph of humoral responses in newborn BALB/c mice immunized with 1 g recombinant HBsAg protein with alum or with 10 g pCMV-S DNA vaccine on the day of birth or 7 days later. Each point represents the group mean (n=10) for anti-HBs titers (total IgG) as determined by end-point dilution ELISA assay.

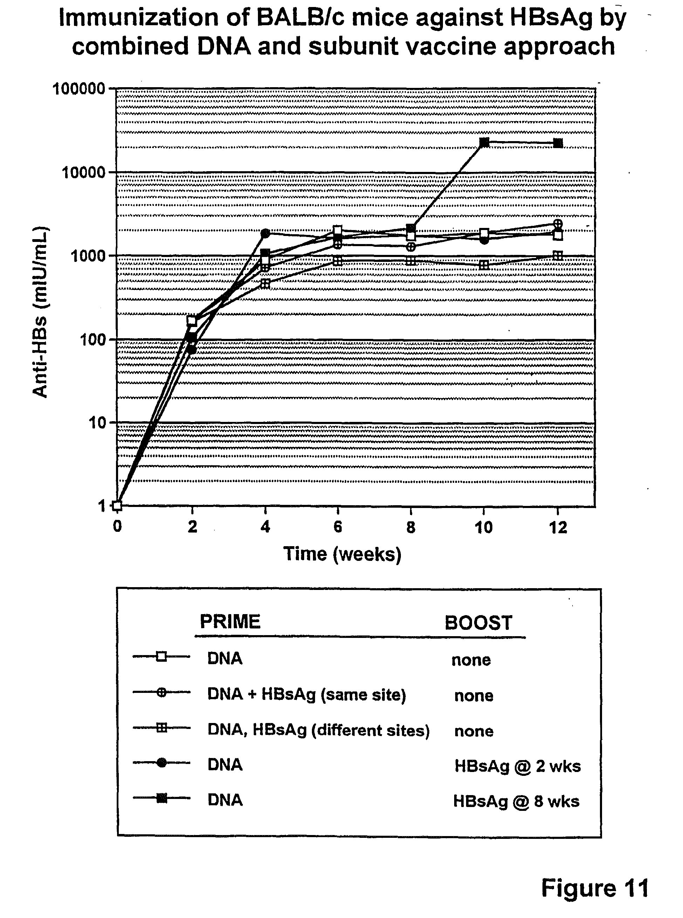

FIG. 11 is a graph of humoral responses in BALB/c mice primed with 10 g HBsAg-expressing DNA vaccine (pCMV-S) and given 2 g recombinant HBsAg protein at the same time in the same or a different muscle or given the HBsAg 2 or 8 weeks later. Each point represents the group mean (n=10) for anti-HBs titers (total IgG) as determined by end-point dilution ELISA assay.

### DESCRIPTION OF THE PREFERRED EMBODIMENTS

It is to be understood that this invention is not limited to the particular methodology, protocols, sequences, models and reagents described as such may, of course, vary. It is also to be understood that the terminology used herein is for the purpose of describing particular embodiments only, and is not intended to limit the scope of the present invention which will be limited only by the appended claims.

All publications mentioned herein are incorporated herein by reference for the purpose of describing and disclosing the oligonucleotides and methodologies which are described in the publications which might be used in connection with the presently described invention.

The binding of DNA to cells has been shown to be similar to a ligand receptor interaction: binding is saturable, competitive, and leads to DNA endocytosis and degradation into oligonucleotides (Bennet, R.M., et al., J. Clin. Invest. 76:2182, 1985). Like DNA, oligodeoxyribonucleotides are able to enter cells in a process which is sequence, temperature, and energy independent (Jaroszewski and Cohen, Ad. Drug Del. Rev. 6:235, 1991). An "oligodeoxyribonucleotide" as used herein is a deoxyribonucleic acid sequence from about 3-50 bases in length. Lymphocyte oligodeoxyribonucleotide uptake has been shown to be regulated by cell activation (Krieg, A.M., et al., Antisense Research and Development 1:161, 1991). The present invention is based on the finding that certain oligonucleotides (ODN) containing at least one unmethylated cytosine-guanine (CpG) dinucleotide activate the immune response.

In one embodiment, the invention provides a method for stimulating an immune response in a subject by administering a therapeutically effective amount of a nucleic acid sequence containing at least one unmethylated CpG. The term "nucleic acid" or "oligonucleotide" refers to a polymeric form of nucleotides at least five bases in length. The nucleotides of the invention can be deoxyribonucleotides, ribonucleotides, or modified forms of either nucleotide. Generally, double-stranded molecules are more stable *in vivo,* while single-stranded molecules have increased activity.

The nucleic acid molecule can include the use of phosphorothioate or phosphorodithioate rather than phosphodiesterase linkages within the backbone of the molecule, or methylphosphorothioate terminal linkages (Krieg, A.M., et al., Antisense and Nucl Acid Drug Dev 6:133-9, 1996; Boggs, R.T., et al., Antisense and Nucl Acid Drug Dev, 7:461-71, 1997). The phosphate backbone modification can occur at the 5' end of the nucleic acid, for example at the first two nucleotides of the 5' end of the nucleic acid. The phosphate backbone modification may occur at the 3' end of the nucleic acid, for example at the last five nucleotides of the 3' end of the nucleic acid. International Patent Application WO 95/26204, entitled "Immune stimulation by phosphorothioate oligonucleotide analogs" reports the nonsequence-specific immunostimulatory effect of phosphorothioate modified oligonucleotides. Nontraditional bases such as inosine and queosine, as well as acetyl-, thio- and similarly modified forms of adenine, cytidine, guanine, thymine, and uridine can also be included, which are not as easily recognized by endogenous endonucleases. Other stabilized nucleic acid molecules include: nonionic DNA analogs, such as alkyl- and aryl-phosphonates (in which the charged oxygen moiety is alkylated). Nucleic acid molecules which contain a diol, such as tetrahyleneglycol or hexaethyleneglycol, at either or both termini are also included. The term "oligonucleotide" includes both single and double-stranded forms of DNA.

A "CpG" or "CpG motif' refers to a nucleic acid having a cytosine followed by a guanine linked by a phosphate bond. The term "methylated CpG" refers to the methylation of the cytosine on the pyrimidine ring, usually occurring the 5-position of the pyrimidine ring. The term "unmethylated CpG" refers to the absence of methylation of the cytosine on the pyrimidine ring. Methylation, partial removal, or removal of an unmethylated CpG motif in an oligonucleotide of the invention is believed to reduce its effect. Methylation or removal of all unmethylated CpG motifs in an oligonucleotide substantially reduces its effect. The effect of methylation or removal of a CpG motif is "substantial" if the effect is similar to that of an oligonucleotide that does not contain a CpG motif.

Preferably the CpG oligonucleotide is in the range of about 8 to 30 bases in size. For use in the instant invention, the nucleic acids can be synthesized *de novo* using any of a number of procedures well known in the art. For example, the b-cyanoethyl phosphoramidite method (Beaucage, S.L., and Caruthers, M.H., Tet. Let. 22:1859, 1981); nucleoside H-phosphonate method (Garegg et al., Tet. Let. 27:4051-4054, 1986; Froehler et al., Nucl. Acid Res. 14:5399-5407, 1986, ; Garegg et al., Tet. Let. 27:4055-4058, 1986, 1Gaffney et al., Tet. Let. 29:2619-2622, 1988). These chemistries can be performed by a variety of automated oligonucleotide synthesizers available in the market. Alternatively, CpG dinucleotides can be produced on a large scale in plasmids, (see Sambrook, T., et al., Molecular Cloning: A Laboratory Manual, Cold Spring Harbor laboratory Press, New York, 1989) which after being administered to a subject are degraded into oligonucleotides. Oligonucleotides can be prepared from existing nucleic acid sequences (*e*.*g*., genomic or cDNA) using known techniques, such as those employing restriction enzymes, exonucleases or endonucleases.

For use *in vivo,* nucleic acids are preferably relatively resistant to degradation (*e*.*g*., via endo-and exo-nucleases). Secondary structures, such as stem loops, can stabilize nucleic acids against degradation. Alternatively, nucleic acid stabilization can be accomplished via phosphate backbone modifications. A preferred stabilized nucleic acid has at least a partial phosphorothioate modified backbone. Phosphorothioates may be synthesized using automated techniques employing either phosphoramidate or H-phosphonate chemistries. Aryl-and alkyl-phosphonates can be made, *e*.*g*., as described in U.S. Patent No. 4,469,863; and alkylphosphotriesters (in which the charged oxygen moiety is alkylated as described in U.S. Patent No. 5,023,243 and European Patent No. 092,574) can be prepared by automated solid phase synthesis using commercially available reagents. Methods for making other DNA backbone modifications and substitutions have been described (Uhlmann, E. and Peyman, A., Chem. Rev. 90:544, 1990; Goodchild, J., Bioconjugate Chem. 1:165,1990).

For administration *in vivo,* nucleic acids may be associated with a molecule that results in higher affinity binding to target cell (*e*.*g*., B-cell, monocytic cell and natural killer (NK) cell) surfaces and/or increased cellular uptake by target cells to form a "nucleic acid delivery complex." Nucleic acids can be ionically or covalently associated with appropriate molecules using techniques which are well known in the art. A variety of coupling or cross-linking agents can be used, *e*.*g*., protein A, carbodiimide, and N-succinimidyl-3-(2-pyridyldithio) propionate (SPDP). Nucleic acids can alternatively be encapsulated in liposomes or virosomes using well-known techniques.

In one embodiment, the nucleic acid sequences useful in the methods of the invention are represented by the formula:

5'N₁X₁CGX₂N₂3' (SEQ ID NO:1)

wherein at least one nucleotide separates consecutive CpGs; X₁ is adenine, guanine, or thymidine; X₂ is cytosine or thymine, N is any nucleotide and N₁ + N₂ is from about 0-26 bases. In a preferred embodiment, N₁ and N₂ do not contain a CCGG quadmer or more than one CGG trimer; and the nucleic acid sequence is from about 8-30 bases in length. However, nucleic acids of any size (even may kb long) can be used in the invention if CpGs are present, as larger nucleic acids are degraded into oligonucleotides inside cells. Preferred synthetic oligonucleotides do not include a CCGG quadmer or more than one CCG or CGG trimer at or near the 5' or 3' terminals and/or the consensus mitogenic CpG motif is not a palindrome. A "palindromic sequence" or "palindrome" means an inverted repeat (*i*.*e*., a sequence such as ABCDEE'D'C'B'A', in which A and A' are bases capable of forming the usual Watson-Crick base pairs.

In another embodiment, the method of the invention includes the use of an oligonucleotide which contains a CpG motif represented by the formula:

5'N₁X₁X₂CGX₃X₄N₂ 3' (SEQ ID NO:2)

wherein at least one nucleotide separates consecutive CpGs; X₁X₂ is selected from the group consisting of GpT, GpG, GpA, ApT and ApA; X₃ X₄ is selected from the group consisting of TpT or CpT; N is any nucleotide and N₁+N₂ is from about 0-26 bases. In a preferred embodiment, N₁ and N₂ do not contain a CCGG quadmer or more than one CCG or CGG trimer. CpG ODN are also preferably in the range of 8 to 30 bases in length, but may be of any size (even many kb long) if sufficient motifs are present, since such larger nucleic acids are degraded into oligonucleotides inside of cells. Preferred synthetic oligonucleotides of this formula do not include a CCGG quadmer or more than one CCG or CGG trimer at or near the 5' and/or 3' terminals and/or the consensus mitogenic CpG motif is not a palindrome. Other CpG oligonucleotides can be assayed for efficacy using methods described herein. An exemplary nucleic acid sequence of the invention is 5'-TCCATGACGTTCCTGACGTT-3' (SEQ ID NO:3).

A prolonged effect can be obtained using stabilized oligonucleotides, where the oligonucleotide incorporates a phosphate backbone modification (*e*.*g*., a phosphorothioate or phosphorodithioate modification). More particularly, the phosphate backbone modification occurs at the 5' end of the nucleic acid for example, at the first two nucleotides of the 5' end of the nucleic acid. Further, the phosphate backbone modification may occur at the 3' end of the nucleic acid for example, at the last five nucleotides of the 3' end of the nucleic acid. Preferred nucleic acids containing an unmethylated CpG have a relatively high stimulation with regard to B cell, monocyte, and/or natural killer cell responses (*e*.*g*., induction of cytokines, proliferative responses, lytic responses, among others).

*Nucleic acids containing an unmethylated CpG can be effective in any mammal, preferably a human. Different nucleic acids containing an unmethylated CpG can cause optimal immune stimulation depending on the mammalian species. Thus an oligonucleotide causing optimal stimulation in humans may not cause optimal stimulation in a mouse. One ofskill in the art can identify the optimal oligonucleotides useful for a particular mammalian species of interest.*

The "stimulation index" is a measure of a CpG ODN to effect an immune response which can be tested in various immune cell assays. The stimulation of the immune response can be assayed by measuring various immune parameters, *e*.*g*., measuring the antibody-forming capacity, number of lymphocyte subpopulations, mixed leukocyte response assay, lymphocyte proliferation assay. The stimulation of the immune response can also be measured in an assay to determine resistance to infection or tumor growth. Methods for measuring a stimulation index are well known to one of skill in the art. For example, one assay is the incorporation of ³H uridine in a murine B cell culture, which has been contacted with a 20µM of oligonucleotide for 20h at 37°C and has been pulsed with 1µCi of³H uridine; and harvested and counted 4h later. The induction of secretion of a particular cytokine can also be used to assess the stimulation index. Without meaning to be bound by theory, for use *in vivo,* for example to treat a subject at risk of exposure to a hepatitis virus, it is important that the CpG ODN be capable of effectively inducing cytokine secretion by monocytic cells and/or Natural Killer (NK) cell lytic activity. In one method, the stimulation index of the CpG ODN with regard to B-cell proliferation is at least about 5, preferably at least about 10, more preferably at least about 15 and most preferably at least about 20, while recognizing that there are differences in the stimulation index among individuals.

The CpG ODN of the invention stimulate cytokine production (*e*.*g*., IL-6, IL-12, IFN-γ, TNF-α and GM-CSF). Exemplary sequences include:
TCCATGTCGCTCCTGATGCT (SEQ ID NO:4),
TCCATGTCGTTCCTGATGCT (SEQ ID NO:5), and
TCGTCGTTTTGTCGTTTTGTCGTT (SEQ ID NO:6).

The CpG ODN of the invention are also useful for stimulating natural killer cell (NK) lytic activity in a subject such as a human. Specific, but nonlimiting examples of such sequences include:
TCGTCGTTGTCGTTGTCGTT (SEQ ID NO:7),
TCOTCGTTTTOTCOTITTGTCGTT (SEQ ID NO:6),
TCGTCGTTGTCGTTTTGTCGTT (SEQ ID NO:8),
GCGTGCGTTGTCGTTGTCGTT (SEQ ID NO:9),
TGTCGTTRGTCGTTTGTCGTT (SEQ ID NO:10),
TGTCGTTGTCGTTGTCGTT (SEQ ID NO:11), and
TCGTCGTCGTCGTT (SEQ ID NO:12).

The nucleic acid sequences of the invention are also useful for stimulating B cell proliferation. Specific, but nonlimiting examples of such sequences include:
TCCTGTCGTTCCTTGTCGTT (SEQ ID NO:13),
TCCTGTCGTTTTTTGTCGTT (SEQ ID NO:14),
TCGTCGCTGTCTGCCCTTCTT (SEQ ID NO:15),
TCGTCGCTGTTGTCGTTTCTT (SEQ ID NO:16),
TCGTCGTTTTGTCGTTTTGTCGTT (SEQ ID NO:6),
TCGTCGTTGTCGTTTTGTCGTT (SEQ ID NO:8) and
TGTCGTTGTCGTTGTCGTT (SEQ ID NO:11).

Preferred CpG ODN can effect at least about 500 pg/ml of TNF-α, 15 pg/ml IFN-γ, 70 pg/ml of GM-CSF 275 pg/ml of IL-6, 200 pg/ml IL-12, depending on the therapeutic indication. These cytokines can be measured by assays well known in the art. The ODNs listed above or other preferred CpG ODN can effect at least about 10%, more preferably at least about 15% and most preferably at least about 20% YAC-1 cell specific lysis or at least about 30%, more preferably at least about 35%, and most preferably at least about 40% 2C11 cell specific lysis, in assays well known in the art.

An "antigenic polypeptide" is any polypeptide that can, under appropriate conditions, induce an immune response. Antigenic polypeptides include, but are not limited to, viral proteins, or fragments thereof. Minor modifications of the primary amino acid sequences of a viral polypeptide may also result in a polypeptide which have substantially equivalent antigenic activity as compared to the unmodified counterpart polypeptide. Such modifications may be deliberate, as by site-directed mutagenesis, or may be spontaneous. All of the polypeptides produced by these modifications are included herein as long as antigenicity still exists. One non-limiting example of an antigenic viral polypeptide is the hepatitis B surface antigen.

The term "substantially purified" as used herein refers to a polypeptide which is substantially free of other proteins, lipids, carbohydrates or other materials with which it is naturally associated. One skilled in the art can purify viral polypeptides using standard techniques for protein purification. The substantially pure polypeptide will yield a single major band on a non-reducing polyacrylamide gel. The purity of the viral polypeptide can also be determined by amino-terminal amino acid sequence analysis.

The invention utilizes polynucleotides encoding the antigenic polypeptides. These polynucleotides include DNA, cDNA and RNA sequences which encode an antigenic polypeptide. Such polynucleotides include naturally occurring, synthetic, and intentionally manipulated polynucleotides. For example, polynucleotide enocing an antigenic polypeptide may be subjected to site-directed mutagenesis, so long as the polypeptide remains antigenic.

The term "polynucleotide" or "nucleic acid sequence" refers to a polymeric form of nucleotides at least 10 bases in length. By "isolated polynucleotide" is meant a polynucleotide that is not immediately contiguous with both of the coding sequences with which it is immediately contiguous (one on the 5' end and one on the 3' end) in the naturally occurring genome of the organism from which it is derived. The term therefore includes, for example, a recombinant DNA which is incorporated into a vector; into an autonomously replicating plasmid or virus; or into the genomic DNA of a prokaryote or eukaryote, or which exists as a separate molecule (e.g. a cDNA) independent of other sequences. The nucleotides of the invention can be ribonucleotides, deoxyribonucleotides, or modified forms of either nucleotide. The term includes single and double forms of DNA.

In the present invention, the polynucleotide sequences encoding an antigenic polypeptide may be inserted into an expression vector. The term "expression vector" refers to a plasmid, virus or other vehicle known in the art that has been manipulated by insertion or incorporation of the genetic sequences encoding the antigenic polypeptide. Polynucleotide sequence which encode the antigenic polypeptide can be operatively linked to expression control sequences. "Operatively linked" refers to a juxtaposition wherein the components so described are in a relationship permitting them to function in their intended manner. An expression control sequence operatively linked to a coding sequence is ligated such that expression of the coding sequence is achieved under conditions compatible with the expression control sequences. As used herein, the term "expression control sequences" refers to nucleic acid sequences that regulate the expression of a nucleic acid sequence to which it is operatively linked. Expression control sequences are operatively linked to a nucleic acid sequence when the expression control sequences control and regulate the transcription and, as appropriate, translation of the nucleic acid sequence. Thus expression control sequences can include appropriate promoters, enhancers, transcription terminators, as start codon (i.e., ATG) in front of a protein-encoding gene, splicing signal for introns, maintenance of the correct reading frame of that gene to permit proper translation of mRNA, and stop codons. The term "control sequences" is intended to included, at a minimum, components whose presence can influence expression, and can also include additional components whose presence is advantageous, for example, leader sequences and fusion partner sequences. Expression control sequences can include a promoter.

By "promoter" is meant minimal sequence sufficient to direct transcription. Also included in the invention are those promoter elements which are sufficient to render promoter-dependent gene expression controllable for cell-type specific, tissue-specific, or inducible by external signals or agents; such elements may be located in the 5' or 3' regions of the gene. Both constitutive and inducible promoters, are included in the invention (see *e*.*g*., Bitter et al., 1987, Methods in Enzymology 153:516-544). Promoters derived from the genome of mammalian cells (*e*.*g*., metallothionein promoter) or from mammalian viruses (*e*.*g*., the retrovirus long terminal repeat; the adenovirus late promoter; the vaccinia virus 7.5K promoter) may be used. Promoters produced by recombinant DNA or synthetic techniques may also be used to provide for transcription of the nucleic acid sequences of the invention.

By "therapeutically effective amount" is meant the quantity of a compound according to the invention necessary to prevent, to cure or at least partially arrest symptoms in a subject. A subject is any mammal, preferably a human. Amounts effective for therapeutic use will, of course, depend on the severity of the disease and the weight and general state of the subject. Typically, dosages used *in vitro* may provide useful guidance in the amounts useful for *in situ* administration of the pharmaceutical composition, and animal models may be used to determine effective dosages for treatment of particular disorders. Various considerations are described, *e*.*g*., in Gilman et al., eds., Goodman And Gilman's: The Pharmacological Bases of Therapeutics, 8th ed., Pergamon Press, 1990; and Remington's Pharmaceutical Sciences, 17th ed., Mack Publishing Co., Easton, Pa., 1990, each of which is herein incorporated by reference.

An oligonucleotide containing at least one unmethylated CpG can be used alone to activate the immune response or can be administered in combination with another adjuvant. An "adjuvant" is any molecule or compound which can stimulate the humoral and/or cellular immune response. For example, when the oligonucleotide containing at least one unmethylated CpG is administered in conjunction with another adjuvant, the oligonucleotide can be administered before, after, and/or simultaneously with the other adjuvant. The oligonucleotide containing at least one unmethylated CpG can have an additional efficacy (*e*.*g*., through antisense or other means) in addition to its ability to activate the immune response.

The invention further provides a method of modulating the level of a cytokine. The term "modulate" envisions the suppression of expression of a particular cytokine when it is overexpressed, or augmentation of the expression of a particular cytokine when it is underexpressed. Modulation of a particular cytokine can occur locally or systemically. It is believed that the CpG oligonucleotides do not directly activate purified NK cells, but rather render them competent to respond to IL-12 with a marked increase in their IFN-γ production. By inducing IL-12 production and the subsequent increased IFN-γ secretion by NK cells, the immunostimulatory nucleic acids also promote a Th1 type immune response. No direct activation of proliferation or cytokine secretion by highly purified T cells has been found. Cytokine profiles determine T cell regulatory and effector functions in immune responses.

Cytokines also play a role in directing the T cell response. Helper (CD4⁺) T cells orchestrate the immune response of mammals through production of soluble factors that act on other immune system cells, including other T cells. Most mature CD4⁺ T helper cells express one of two cytokine profiles: Th1 or Th2. Th1 cells secrete IL-2, IL-3, IFN-γ, TNF-β, GM-CSF and high levels of TNF-α. Th2 cells express IL-3, IL-4, IL-5, IL-6, IL-9, IL-10, IL-13, GM-CSF and low levels of TNF-α. The Th1 subset promotes delayed-type hypersensitivity, cell-mediated immunity, and immunoglobulin class switching to IgG₂ₐ.

The Th2 subset induces humoral immunity by activating B cells, promoting antibody production, and inducing class switching to IgG₁ and IgE.

Several factors have been shown to influence commitment to Th1 or Th2 profiles. The best characterized regulators are cytokines. IL-12 and IFN-γ are positive Th1 and negative Th2 regulators. IL-12 promotes IFN-γ production, and IFN-γ provides positive feedback for IL-12. IL-4 and IL-10 appear to be required for the establishment of the Th2 cytokine profile and to down-regulate Th1 cytokine production; the effects of IL-4 are in some cases dominant over those of IL-12. IL-13 was shown to inhibit expression of inflammatory cytokines, including IL-12 and TNF-α by LPS-induced monocytes, in a way similar to IL-4. The IL-12 p40 homodimer binds to the IL-12 receptor and antagonizes IL-12 biological activity; thus it blocks the pro-Th1 effects of IL-12.

This invention further provides administering to a subject having or at risk of having an virally mediated disorder, a therapeutically effective dose of a pharmaceutical composition containing the compounds of the present invention and a pharmaceutically acceptable carrier. "Administering" the pharmaceutical composition of the present invention may be accomplished by any means known to the skilled artisan.

The pharmaceutical compositions according to the invention are in general administered topically, intravenously, orally, parenterally or as implants, and even rectal use is possible in principle. Suitable solid or liquid pharmaceutical preparation forms are, for example, granules, powders, tablets, coated tablets, (micro)capsules, suppositories, syrups, emulsions, suspensions, creams, aerosols, drops or injectable solution in ampule form and also preparations with protracted release of active compounds, in whose preparation excipients and additives and/or auxiliaries such as disintegrants, binders, coating agents, swelling agents, lubricants, flavorings, sweeteners or solubilizers are customarily used as described above. The pharmaceutical compositions are suitable for use in a variety of drug delivery systems. For a brief review of present methods for drug delivery, see Langer, Science 249:1527-1533, 1990, which is incorporated herein by reference.

The pharmaceutical compositions are preferably prepared and administered in dose units. Solid dose units are tablets, capsules and suppositories. For treatment of a patient, depending on activity of the compound, manner of administration, nature and severity of the disorder, age and body weight of the patient, different daily doses are necessary. Under certain circumstances, however, higher or lower daily doses may be appropriate. The administration of the daily dose can be carried out both by single administration in the form of an individual dose unit or else several smaller dose units and also by multiple administration of subdivided doses at specific intervals.

The pharmaceutical compositions according to the invention may be administered locally or systemically. By "therapeutically effective dose" is meant the quantity of a compound according to the invention necessary to prevent, to cure or at least partially arrest the symptoms of the disorder and its complications. Amounts effective for this use will, of course, depend on the severity of the disease and the weight and general state of the patient. Typically, dosages used *in vitro* may provide useful guidance in the amounts useful for *in situ* administration of the pharmaceutical composition, and animal models may be used to determine effective dosages for treatment of particular disorders. Various considerations are described, *e*.*g*., in Gilman et al., eds., Goodman And Gilman's: The Pharmacological Bases of Therapeutics, 8th ed., Pergamon Press, 1990; and Remington's Pharmaceutical Sciences, 17th ed., Mack Publishing Co., Easton, Pa., 1990, each of which is herein incorporated by reference.

The following examples are intended to illustrate but not to limit the invention in any manner, shape, or form, either explicitly or implicitly. While they are typical of those that might be used, other procedures, methodologies, or techniques known to those skilled in the art may alternatively be used.

### EXAMPLES

Here we evaluate the use of CpG ODN as an adjuvant for immunization of mice against hepatitis B virus surface antigen (HbsAg) given as a recombinant protein or expressed *in vivo* from a DNA vaccine.

Compared with the recombinant protein vaccine alone, addition of either CpG ODN or alum alone resulted in a 10-100 fold increase in the level of antibodies against HBsAg (anti-HBs). However when used together, these two adjuvants resulted in 500-1000 times higher levels of anti-HBs, indicating a strong synergistic response. Immunization with HBsAg alone or with alum resulted in a strong Th2-type response with almost all IgG being of the IgG1 isotype. CpG ODN induced a high proportion of IgG2a, indicative of a Th1-type response, even in the presence of alum.

DNA "vaccines" also induce potent Th1-type immune responses and this is likely due in large part to the presence of CpG motifs in the bacterially-derived plasmid DNA. We show here that responses are further augmented by the addition of CpG ODN to the DNA vaccine. The DNA vaccine but not the protein subunit vaccine was able to induce anti-HBs in mice injected on the day of birth. A combination approach of DNA prime and protein boost appears to be particularly effective for vaccination purposes, although a sufficient period (>2 weeks) must elapse before a boosting response is seen. These studies demonstrate that the addition of CpG ODN to protein or DNA vaccines is a valid new adjuvant approach to improve efficacy.

### MATERIALS AND METHODS

### Animals

Experiments on adult mice were carried out using female BALB/c (H-2^{d}, good responder), C57BL/6 (B 10, H-2^{b}, fair responder) and B10.S (H-2^{S} ,MHC-restricted hypo-responder to HBsAg) mice (Charles River, Montreal, QC) at 6-8 weeks of age. Mice with class II MHC deficiency (C2D, H-2^{b}, GenPharm, Mountain View, CA) due to gene knockout were used as a model of a non-responder.

Newborn mice were obtained through breeding male and female BALB/c mice (Charles River) in our own animal facility (Loeb Research Institute, Ottawa Civic Hospital, Ottawa, ON). Pregnant females were monitored daily to ensure accurate recording of the date of birth. Both male and female neonates were used for immunization.

### HbsAg subunit vaccination of mice

The subunit vaccine consisted of HBsAg (ay subtype) which had been produced as a recombinant protein in yeast cells (Medix Biotech #ABH0905). This was diluted in saline for use without adjuvant. HBsAg was also formulated with alum and/or CpG ODN as adjuvant. HBsAg protein was mixed with aluminum hydroxide (Alhydrogel 85, [Al₂O₃], Superfos Biosector, Vedbaek, Denmark) in the same ratio of 25 mg Al³⁺ per mg protein as used in the commercial vaccines (i.e., 2.512% Al₂O₃ per g HBsAg). The protein and alum were mixed with a vortex and then left on ice for at least 30 minutes prior to use to allow the protein to adsorb onto the Al₂O₃. This solution was mixed again immediately prior to injection by drawing up into the syringe 3-5 times,

For groups treated with CpG ODN, an appropriate volume of synthetic oligodeoxynucleotide (ODN #1826) of the sequence TCCATGACGTTCCTGACGTT synthesized with a phosphorothioate backbone (Oligos Etc. & Oligo Therapeutics, Wilsonville, OR) was added alone or with alum to HBsAg on the day of injection. Adult mice received a single intramuscular (IM) injection into the left tibialis anterior (TA) muscle of 1 or 2 g HBsAg, without or with adjuvant (alum and/or CpG ODN), in 50 l vehicle. When CpG DNA was added, each animal received a total of 1,10,100 or 500 g ODN. Newborn mice were immunized within 24 hours of birth or 7 days after birth by bilateral injection of a total of 1 g HBsAg into the posterior thigh muscles (2 x 101 @ 0.05 mg/ml). All injections were carried out with a 0.3 ml insulin syringe which has a fused 29G needle (Becton Dickenson, Franklin Lakes, NJ). For injection of adults, the needle was fitted with a collar of polyethylene (PE) tubing to limit penetration of the needle to about 3 mm. All intramuscular injections were carried out through the skin (unshaved) and under general anesthesia (Halothane, Halocarbon Laboratories, River Edge, NJ).

### DNA-based immunization of mice

Mice were immunized against HBsAg using plasmid constructs encoding the major protein (S) of the HBV envelope. The plasmid pCP10, containing two copies of the HBV genome (*ayw* subtype; GeneBank reference HPBAYW) as a head-to-tail fusion (Dubois *et al.,* 1980), was the source of the envelope coding sequences and the 3' untranslated sequences which include the viral polyadenylation signal. A 1.9 kb *Xho*I*-Bgl*II restriction fragment from pCP10 (containing the S coding sequences) was cloned into the corresponding sites of a modified p Bluescript SK vector containing extra restriction sites in the polylinker (kindly provided by Dr. Shahragim Tajbakhsh, Pasteur Institute). A *Kpn*I*-Bss*HII restriction fragment was then removed and cloned into the pRc/CMV expression vector (Invitrogen) using the *Kpn*I site of the polylinker and the *Bss*HII site within the neomycin gene of the vector. This cloning step places the envelope sequences under the control of the CMV promoter and removes the bovine growth hormone polyadenylation sequences, the f1 origin, the SV40 promoter and origin, and most of the neomycin gene. An SV40 polyadenylation signal from the pRc/CMV vector is found after the transcribed HBV sequences. This construct has been described previously (Davis *et al.,* 1993) and is designated here as pCMV-S.

DNA was purified on Qiagen anion-exchange chromatography columns (Qiagen GmbH, Hilden, Germany). This method, which yields predominantly supercoiled, double-stranded, closed circular DNA, results in virtually no contamination with chromosomal DNA, RNA or protein and very low contamination with endotoxin. The DNA was resuspended in sterile saline (0.15M NaCl, BDH) and the concentration of DNA was calculated based on absorbance of ultraviolet light (OD 260). The final concentration was adjusted to 0.1 to 1 mg/ml and the DNA solutions were stored at -20C until required for injection.

Direct gene transfer in adult mice was carried out by unilateral or bilateral IM injection into the TA muscle of DNA in 501 such that each animal received a total of 1,10 or 100 g DNA. Newborn mice received a total of 10 g DNA by bilateral injection into the posterior thigh muscles (2 x 10 1 @ 0.5 mg/ml). Injections were with a 0.3 ml insulin syringe which has a fused 29G needle, and for injection of adults the needle was fitted with a collar of PE tubing to limit penetration of the needle to about 2 mm. All intramuscular injections were carried out (through shaved skin for adults) under general anesthesia (Halothane)

### Experimental groups

### Comparison of alum and CpG ODN as adjuvant. with HBsAg subunit vaccine

Six groups of adult BALB/c mice (n=10) were injected with 1 g HBsAg (i) alone, (ii) mixed with alum, (iii) mixed with 100 g CpG ODN, or (iv, v, vi) mixed with both alum and 10, 100 or 500 g CpG ODN. These mice were bled at 1, 2, 3 and 4 weeks after immunization and the plasma was assayed for anti-HBs. Groups of adult C57BL/6 (B10), B10.S and C2D mice (n=5) were injected 1 g HBsAg (i) alone, (ii) mixed with alum, (iii, iv, v) mixed with 1, 10 or 100 g CpG ODN, or (vi, vii) mixed with both alum and 10 or 100 g CpG ODN. Each animal was boosted by the identical procedure at 6 weeks. The mice were bled at 1, 2, 4, 6 and 8 weeks after immunization and the plasma was assayed for anti-HBs.

### Use of CpG ODN as adjuvant. with HBsAg-expressing DNA vaccine

Three groups of adult BALB/c mice (n=10) were injected with a total of 10 g pCMV-S DNA alone or with 100 or 500 g CpG ODN added, divided between two injection sites. Groups of hypo-responder (B10.S) and congenic (B10) mice (n=10) were immunized with 50 g pCMV-S DNA divided between two sites.

### Immunization of neonates with subunit or DNA vaccine

Groups of newborn BALB/c mice (n=10) aged <24 hours or 7 days were injected with a total of 1 g HBsAg with alum or with 10 g pCMV-S DNA. Plasma was obtained at 4, 8, 12 and 16 weeks for assay of anti-HBs.

### Combined DNA prime with protein boost

Five groups of BALB/c mice (n=10) were first immunized with a single injection into the left TA of 10 g pCMV-S, and received (i) no other treatment, or received 2 g pure HBsAg (no adjuvant) (ii) at the same time at the same site, (iii) at the same time and at a different site (left quadriceps), (iii) 2 weeks later at the different site or (iv) 8 weeks later at the different site. Mice were bled at 1,2,4,6, 8,12,20 and 24 weeks.

### Evaluation of immune response to HbsAg

Heparinized blood was collected by retrobulbar puncture of lightly anaesthetized mice as described elsewhere (Michel *et al*. , 1995). Plasma was recovered by centrifugation (7 min @ 13,000 rpm). Antibodies specific to HBsAg in plasma were detected and quantified by end-point dilution ELISA assay (in triplicate) on individual samples. Ten-fold serial dilutions of plasma were first added to 96-well microtiter plates with a solid phase consisting of plasma-derived HBsAg particles (100 1/well of HBsAg *ay* subtype at 1 g/ml, coated overnight at RT) and incubated for 1 hr at 37C. The bound antibodies were then detected by incubation for 1 hr at 37C with HRP-conjugated goat anti-mouse IgG, IgM, IgG1 or IgG2a (1:4000 in PBS-Tween, 10% FCS; 100 l/well, Southern Biotechnology Inc., Birmingham, AL), followed by incubation with OPD solution (1001/well, Sigma, St. Louis, MO) for 30 minutes at RT in the dark. The reaction was stopped by the addition of sulfuric acid (501 of 4N H₂SO₄). End-point titers were defined as the highest plasma dilution that resulted in an absorbance value (OD 450) two times greater than that of non-immune plasma with a cut-off value of 0.05. Anti-HBs titers were expressed as group means of individual animal values, which were themselves the average of triplicate assays.

### RESULTS

### CpG ODN versus alum as adjuvant for HBV subunit vaccine

### Strength and kinetics of humoral response

Immunization of BALB/c mice with HBsAg alone elicited only low titers of anti-HBs (<100) by 4 weeks. These titers were 10-fold higher with the addition of alum as adjuvant, 60-fold higher with CpG ODN and more than 500-fold higher with both alum and CpG ODN (FIG. 1). When combined with alum, there is a dose-response for CpG ODN with the best results being obtained with an intermediate dose (100 g) and similar somewhat poorer results being obtained with lower and higher doses (10 or 500 g) (FIG. 2). Nevertheless, all doses of CpG ODN greatly improved the titers compared to alum alone.

In the C57BL/6 strain of mouse, antibody titers 4 weeks after HBsAg prime were about 10-times lower than those seen in BALB/c mice, but when boosted at 6 weeks rose to similar levels within 2 weeks. When used alone, alum and CpG ODN (100 g) each augmented the humoral response about 100-fold but when used together increased titers about 1000-fold. A dose-response for CpG ODN was also noted in these mice (in the absence of alum) with 100 g being superior to either 10 or 1 g, although all doses had an adjuvant effect (FIG. 4).

In the hypo-responder B10.S mice no anti-HBs antibodies were detected with HBsAg alone and low titers were obtained if either CpG ODN or alum are added. The use of alum and CpG together gives the best result although the synergy is less evident than in the BALB/c and C57BL/6 mice (FIG. 5). Non-responder (C2D) mice have no detectable anti-HBs after immunization with HBsAg alone. There are low levels of IgM with addition of alum and this is increased 4-fold with further addition of CpG ODN. There is essentially no detectable IgG after injection of HBsAg + alum, but low titers with both alum and CpG ODN (FIG. 6). Treatment with CpG DNA was well tolerated by all mice, even those receiving the 500 g dose. There was no apparent ruffling of fur, diarrhea or other signs of toxicity.

### Th1 versus Th2 responses

Immunization with either HBsAg alone or with alum induces a predominantly Th2-type humoral response with almost all (>99%) antibodies being of the IgG1 isotype. CpG ODN induces significantly more IgG2a antibodies, which are indicative of a Th1-type response, although IgG1 still predominate. Remarkably, the combination of alum and CpG ODN induces about ten-times more IgG2a than IgG1, indicating a real shift from Th2 to Th1 (FIG. 7).

### CpG ODN as adjuvant to HBV DNA vaccine

DNA vaccines induced higher levels of anti-HBs more rapidly than did HBsAg, even when alum was included (compare FIG.s 1 and 8). Addition of CpG ODN to the pCMV-S DNA vaccine increased anti-HBs titers a further five-fold by 4 weeks. The 500 g dose was slightly better than the 100 g dose (FIG. 8). The DNA vaccine was also superior to the HBsAg subunit vaccine in hypo-responder mice. A single injection of DNA induced earlier appearance of anti-HBs, and these reached higher titers than with two doses of protein given at 0 and 4 weeks (FIG. 9).

### Antigen versus DNA-based immunization of neonates

Mice immunized with HBsAg plus alum on the day of birth had no detectable anti-HBs even up to 16 weeks later. In contrast, those injected with the DNA vaccine had low levels of anti-HBs by 4 weeks and a good titer (10³) by 16 weeks. Immunization of 7 day old mice with either DNA or protein induced anti-HBs, although these appeared much earlier and were much higher with the DNA vaccine. In fact, the DNA vaccine at one day was superior to the protein vaccine given at 7 days (FIG. 10).

### Combined DNA- and antigen-based immunization

Co-administration of pure recombinant HBsAg and the DNA vaccine at either the same or different sites did not significantly improve titers of anti-HBs over those induced by the DNA vaccine alone. Nor was a boosting response seen when the HBsAg protein was given two weeks after the DNA vaccine. However, administration of the protein vaccine 8 weeks after the DNA vaccine gave a strong boosting response with titers increasing more than 10-fold over those with the DNA vaccine alone (FIG. 11).

### DISCUSSION

### CpG ODN versus alum as adjuvant with HBV subunit vaccine in mice

CpG ODN is as good as or superior to alum when each is used alone as adjuvant with the HBsAg subunit vaccine in mice, This indicates that CpG ODN could be used to replace alum in vaccine formulations, which could be desirable to avoid associated side-effects due to local irritation in the muscle. Furthermore, for certain live-attenuated or multivalent vaccines, it is not possible to use alum which through chemical interactions interferes with the efficacy of the vaccine. This should not occur with CpG ODN.

Of even greater interest is the strong synergistic response when CpG ODN and alum are used together to adjuvant the HBsAg subunit vaccine. In humans, this could result in a much higher proportion of individuals attaining protective titers of anti-HBs after two or possibly even one dose of vaccine. Furthermore, protective titers should be reached more quickly and this would be beneficial for immunization in endemic areas. There is a fairly weak dose response to CpG ODN whether or not alum is present, indicating that a wide range of CpG ODN could be useful to adjuvant vaccines in humans.

### CpG ODN induces Th1 response even in presence of alum

Aluminum hydroxide (alum) is currently the only adjuvant approved for human use. An important disadvantage of alum is that it induces a Th2- rather than a Th1-type immune response, and this may interfere with induction of CTL. Indeed, in mice immunized with recombinant HBsAg, the addition of alum selectively blocked activation of CD8⁺ CTL (Schirmbeck *et al.,* 1994). Although not essential for protective immunity against HBV, CTL may nevertheless play an important role. For example, a lack of HBV-specific CTL is thought to contribute to the chronic carrier state. In contrast, one of the primary advantages of CpG DNA over alum as an adjuvant is the Th1-bias of the responses and thus the possibility to induce CTL. A striking finding from the present study is that CpG can completely counteract the Th2-bias of alum when the two adjuvants are delivered together. This could allow one to capitalize on the strong synergistic action of the two adjuvants on the humoral response while still allowing CTL.

The use of alum has been linked to Th2-type diseases. The much higher prevalence of asthma (another Th2-type disease) in more highly developed nations may be linked to the high hygiene level and rapid treatment of childhood infections (Cookson and Moffatt, 1997). Early exposure to bacterial DNA (and immunostimulatory CpG motifs) pushes the immune system away from Th2- and towards a Th1-type response and this may account for the lower incidence of asthma in less developed countries, where there is a much higher frequency of upper respiratory infections during childhood. Addition of CpG ODN as adjuvant to all pediatric vaccines could re-establish a Th1-type response thereby reducing the incidence of asthma.

### CpG dinucleotides and DNA vaccines

More recently however, it has been shown that the presence of unmethylated CpG motifs in the DNA vaccines is essential for the induction of immune responses against the antigen, which is expressed only in very small quantities (Sato *et al*. , 1996). As such, the DNA vaccine provides its own adjuvant in the form of CpG DNA. Since single-stranded but not double-stranded DNA can induce immunostimulation *in vitro* (Krieg *et al.,* unpublished observation), the CpG adjuvant effect of DNA vaccines *in vivo* is likely due to oligonucleotides resulting from plasmid degradation by nucleases. Only a small portion of the plasmid DNA injected into a muscle actually enters a myofiber and is expressed, the majority of the plasmids is degraded in the extracellular space.

### CpG and HBV vaccines for human use

### Prophylactic vaccine

Fewer than 20% of healthy individuals attain protective levels of anti-HBs (10 mIU/ml) after a single dose of subunit HBV vaccine and only 60-70% reach this level after two doses. Thus, three doses (usually given at 0, 1 and 6 months) are required to seroconvert >90% of vaccinated individuals. The three dose regime is frequently not completed owing to poor patient compliance, and in endemic areas, protective levels may not be induced quickly enough. Thus there is a need for a prophylactic vaccine that can induce protective immunity more quickly and with fewer doses. This might be possible with the addition of CpG ODN as an adjuvant to the subunit vaccine. Another possibility is an HBsAg-expressing DNA vaccine, which could be optimized by addition of CpG dinucleotide motifs. DNA vaccines would offer additional advantages such as relatively low cost and ease of manufacturing, and heat-stability which circumvents the requirement for a cold-chain.

Neonates bom in endemic areas require particularly rapid induction of strong HHV-specific immunity owing to the high rate of chronicity resulting from infection at a young age. Without immunoprophylaxis, 70-90% of infants bom to mothers positive for both HBsAg and the e antigen (HBeAg) become infected and almost all of these become chronic carriers (Stevens *et al*. , 1987). Even when vaccinated with a four dose regime of the HBV subunit vaccine commencing on the day of birth, 20% of such infants became chronically infected and this was reduced to only 15% if they were also given HBV-specific immunoglobulin (Chen *et al*. , 1996). Subunit or DNA vaccines with CpG adjuvant should reduce this further owing to a more rapid appearance and higher titers of anti-HBs antibodies and the induction of HBV-specific CTL, which could help clear virus from the liver of babies infected *in utero*, and which likely account for most of the failures with neonatal vaccination. DNA vaccines could be particularly effective if coupled with a protein boost.

### Non-responders and hypo-responders

Between 5 and 10% of individuals are non-responders or hypo-responders to the subunit HBsAg vaccine. This may be MHC-restricted (Kruskall *et al*., 1992) and is thought to result from a failure to recognize T-helper epitopes. In certain immunocompromised individuals (e.g., kidney dialysis patients, alcoholics) the rate of non-response can approach 50%. In the present study, alum plus CpG ODN gave higher anti-HBs titers than alum alone in a strain of mice which has MHC-restricted hypo-responsiveness to HBsAg, thought to result in a failure to recognize T-helper epitopes. CpG ODN also overcame non-response in mice genetically incapable of providing T-help owing to an absence of class II MHC (Milich, 1988). These results support the *in vitro* finding that CpG ODN drives the T cell independent activation of B cells. Use of CpG DNA as an adjuvant may increase the response rate to HBsAg in humans. A link between MHC phenotype and non-responsiveness to HBsAg has been demonstrated in humans (Kruskall *et al.,* 1992).

### Chronic carriers of HBY

HBV chronicity results in 10-15% of individuals infected as adolescents or adults, but 90-95% for those infected (either vertically or horizontally) as infants. HBV chronicity eventually leads to cirrhosis and increased risk of hepatocellular carcinoma and an estimated one million people die each year from HBV-related liver disease. Persistent HBV infection of the liver results when acute infection fails to launch an appropriate immune response to clear the virus. Such chronic carriers have circulating HBsAg and HBV core antigen (HBcAg/HBeAg) without specific immunity. It is thought that the absence of HBV-specific CTL may contribute to the establishment and maintenance of the chronic carrier state. Indeed, many previously infected individuals, even years after clinical and serological recovery, have traces of HBV in their blood and HBV-specific CTL that express activation markers indicative of recent contact with antigen (Rehermann *et al.,* 1996). These results suggest that sterilizing immunity may not occur after HBV infection and that chronic activation of CTL is responsible for keeping the virus under control.

There is currently no cure for the HBV chronic infection. Interferon is used currently but this cures only 10-20% of treated individuals (Niederau *et al*., 1996). Anti-viral drugs (e.g., lamivudine) can reduce circulating virus to undetectable levels, however these return to pretreatment levels if the drug is stopped. Each of these types of treatment is also expensive and has certain undesirable side-effects.

The possibility to induce a strong Th 1-type response with CpG ODN added to a subunit vaccine may help overcome the chronic carrier state. For this application, it might be desirable to include additional B and T cell epitopes encoded by other domains of the HBV envelope protein (e.g., pre-S1 and pre-S2). Since the pre-S1 polypeptide may prevent secretion, it might be desirable to encode a truncated version of this such as that described by Li *et al*. (1994) with only amino acids 21-47 which include the hepatocyte receptor-binding site and induce anti-preS 1 immune responses yet still maintain particle secretion. Repeated doses of a subunit vaccine containing the middle HBV envelope protein (preS2 + S) reduced viral replication in 50% of vaccinated chronic carriers (Pol *et al*. , 1993). Addition of CpG ODN would presumably improve these results through its strong Th1 bias.

A DNA vaccine might also prove very effective as a therapeutic vaccine for HBV chronic carriers. We have previously shown that an HBsAg-expressing DNA vaccine could break tolerance to HBsAg in transgenic mice expressing HBsAg in the liver from before birth (Mancini *et al*. , 1996). This response appears to be mediated by T cells via a non-lytic mechanism. Addition of CpG dinucleotide motifs that preferentially induce Th1 cytokines and strong CTL responses could further improve DNA vaccines for application to HBV chronic carriers.

### CONCLUSION

ODN containing CpG dinucleotides, in the proper base context to cause immune activation, are useful as an adjuvant to protein vaccines (whole pathogen or subunit). The CpG ODN could be used alone or in combination with alum. Used alone, it will allow the possibility to adjuvant vaccines that cannot be mixed with alum (e.g., live attenuated pathogens, multivalent vaccines). Used together, it will capitalize on the synergistic effect to induce very potent immune responses, yet still maintain the Th1 bias of CpG DNA. CpG dinucleotides also act to adjuvant DNA vaccines. Additional CpG given as ODN or cloned into the plasmid vector could further augment immune responses.

With respect to vaccines against HBV, CpG ODN could be added as an adjuvant to recombinant HBsAg subunit vaccines, either alone or in combination with alum, or can be cloned into an HBsAg-expressing DNA vaccine. These improved vaccines can (i) induce higher titers more quickly and reduce the number of doses required to induce protective immunity from three to two, (ii) overcome hypo- or non- responsiveness to HBsAg, (iii) control the chronic carrier state through induction of CTL, and (iv) induce rapid and stronger immunity in neonates in HBV endemic areas.

In one aspect, the present invention provides a method of inducing an immune response in a subject, said method comprising administering to the subject a therapeutically effective amount of nucleic acid encoding an antigenic polypeptide, and a therapeutically effective amount of an oligonucleotide containing at least one unmethylated CpG dinucleotide.

In another aspect, the present invention provides a method for treating a subject having or at risk of having a viral-mediated disorder, comprising administering to the subject a therapeutically effective amount of a nucleic acid encoding an antigenic polypeptide and an effective amount of an oligonucleotide containing at least one unmethylated CpG dinucleotide.

In a further aspect, the present invention provides a method for treating a subject having or at risk or having a chronic viral infection, said method comprising administering to the subject an effective amount of a nucleic acid encoding an antigenic polypeptide and an effective amount of an oligonucleotide containing at least one unmethylated CpG dinucleotide.

The following are options in these last three aspects of the invention:
the oligonucleotide can be from 8-30 bases in length;
the subject can be human;
said nucleic acid encoding an antigenic protein and effective amount of an oligonucleotide containing at least one unmethylated CpG dinucleotide can be administered in a vector;
   said nucleic acid encoding an antigenic protein can encode a viral antigen, a hepatitis viral antigen, or a hepatitis B virus surface antigen;
   the method can further comprise administering an adjuvant, which can be aluminium hydroxide;
   the oligonucleotide can have the formula 5'N₁X₁CGX₂N₂3' (SEQ ID NO: 1), wherein at least one nucleotide separates consecutive CpGs, X₁ is adenine, guanine, or thymidine, X₂ is cytosine or thymine, N is any nucleotide and N₁ + N₂ is from about 0-26 bases;
   the oligonucleotide can have the formula 5'N₁X₁CGX₂N₂3' (SEQ ID NO: 1) as defined above, but N₁ and N₂ do not contain a CCGG quadmer or more than one CCG trimer, and the nucleic acid sequence is from about 8-30 bases in length;
   the oligonucleotide can have the formula 5'N₁X₁X₂CGX₃X₄N₂3' (SEQ ID NO: 2), wherein at least one nucleotide separates consecutive CpGs, X₁X₂ is selected from the group consisting of GpT, GpG, GpA, ApT and ApA, X₃X₄ is selected from the group consisting of TpT and CpT, N is any nucleotide, and N₁ + N₂ is from about 0-26 bases;
   the oligonucleotide can have the formula 5'N₁X₁X₂CGX₃X₄N₂3' (SEQ ID NO: 2) as defined above, but N₁ and N₂ do not contain a CCGG quadmer or more than one CCG or CGG trimer, and the nucleic acid sequence is from about 8-30 bases in length;
   the oligonucleotide can be 5'-TCCATGACGTTCCTGACGTT-3' (SEQ ID NO: 3); and,
   said method can further comprise administering to the subject a therapeutically effective amount of the antigenic polypeptide.

In a yet further aspect, the present invention provides a pharmaceutical composition comprising an immunostimulatory CpG oligonucleotide and a nucleic acid encoding an antigenic protein in a pharmaceutically acceptable carrier.

### REFERENCES

Chen D.S, et al. (1996). Cancer Causes & Control 7: 305-311.
Cookson, W.O.C.M. & Moffatt, M.F. (1997). Science 275: 41-42.
Cowdery, J.S. et al. (1996). J. Immunol. 156: 4570.
Davis, H.L. et al. (1993). Human Molec. Genet. 2: 1847-1851.
Davis, H.L. et al. (1995). Human Gene Ther. 6: 1447-1456.
Davis, H.L. et al. (1996). Proc. Natl. Acad. Sci. USA 93: 7213-7218.
Davis, H.L. et al. (1996). Vaccine 14: 910-915.
Davis, H.L. et al. (1997). Gene Ther. (in press).
Davis, H.L. & Brazolot Millan, C.L. (1997). Blood Cell Biochem. (in press)
Donnelly, J.J. et al. (1996). Life Sciences. 60:163-172.
Dubois, M.-F. et al. (1980). Proc. Natl. Acad. Sci. USA 77: 4549-4553,
Ellis, R. W. (Ed.) (1993). Hepatitis B Vaccines in Clinical Practise. New York: Marcel-Dekker.
Halpern, M.D. et al. (1996). Cell. Immunol. 167: 72.
Klinman, D.M. et al. (1996). Proc. Natl. Acad. Sci. USA 93: 2879-2883.
Krieg, A.M. et al. (1995). Nature 374: 546-549.
Kruskall, M.S. et al. (1992). J. Exp. Med. 175: 495-502.
Li et al. , (1994). J. Gen. Virol. 75:3673-3677.
Mancini, M. et al. (1996). Proc. Natl. Acad. Sci. USA 93: 12496-12501.
Michel, M.-L. et al. (1984) Proc. Natl. Acad. Sci. USA 81: 7708-7712.
Michel, M.-L. et al. (1995). Proc. Natl. Acad. Sci. USA 92: 5307-5311.
Milich, D.R. (1988). Immunol. Today 9: 380-386.
Niederau et al. (1996). New Eng. J. Med. 334: 1422-1427.
Pol, S. et al. (1993). C. R. Acad. Sci. (Paris) 316: 688-691.
Rehermann, B. et al. (1996). Nature Med. 2: 1104-1108.
Sato, Y., et al. (1996). Science 273: 352-354.
Schirmbeck, R. et al. (1994). J. Immunol. 152: 1110-1119.
Stevens, C.E. et al. (1987). J. Am. Med. Assoc. 257: 2612-2616.
Vogel, F.R. & Sarver, N. (1995). Clin. Microbial. Rev. 8: 406-410.

## Claims

1. An oligonucleotide containing at least one unmethylated CpG dinucleotide, for use in a method of inducing an immune response in a subject, wherein a therapeutically effective amount of said oligonucleotide and a therapeutically effective amount of a nucleic acid encoding an antigenic polypeptide are to be administered to the subject.

2. An oligonucleotide containing at least one unmethylated CpG dinucleotide, for use in a method for treating a subject having or at risk of having a viral-mediated disorder, wherein an effective amount of said oligonucleotide and a therapeutically effective amount of a nucleic acid encoding an antigenic polypeptide are to be administered to the subject.

3. An oligonucleotide containing at least one unmethylated CpG dinucleotide, for use in a method for treating a subject having or at risk of having a chronic viral infection, wherein an effective amount of said oligonucleotide and an effective amount of a nucleic acid encoding an antigenic polypeptide are to be administered to the subject.

4. An oligonucleotide as claimed in claim 1, 2 or 3, from 8-30 bases in length.

5. An oligonucleotide as claimed in claim 1, 2 or 3, wherein the subject is human.

6. An oligonucleotide as claimed in claim 1 or 2, wherein said nucleic acid encoding an antigenic protein and effective amount of an oligonucleotide containing at least one unmethylated CpG dinucleotide are administered in a vector.

7. An oligonucleotide as claimed in claim 1, 2 or 3, wherein said nucleic acid encoding an antigenic protein encodes a viral antigen, a hepatitis viral antigen, or a hepatitis B virus surface antigen.

8. An oligonucleotide as claimed in claim 1, 2 or 3, wherein said method further comprises administering an adjuvant, wherein the adjuvant, optionally, is aluminium hydroxide.

9. An oligonucleotide as claimed in claim 1, 2 or 3, wherein the oligonucleotide has a formula:
5'N₁X₁CGX₂N₂3' (SEQ ID NO: 1)
wherein at least one nucleotide separates consecutive CpGs; X₁ is adenine, guanine, or thymidine; X₂ is cytosine or thymine; N is any nucleotide; and N₁ + N₂ is from about 0-26 bases.

10. An oligonucleotide as claimed in claim 9, wherein N₁ and N₂ do not contain CCGG quadmer or more than one CGG trimer; and the nucleic acid sequence is from about 8-30 bases in length.

11. An oligonucleotide as claimed in claim 1, 2 or 3, wherein the oligonucleotide has a formula:
5'N₁X₁X₂CGX₃X₄N₂3' (SEQ ID NO: 2)
wherein at least one nucleotide separates consecutive CpGs; X₁X₂ is selected from the group consisting of GpT, GpG, GpA, ApT and ApA; X₃X₄ is selected from the group consisting of TpT and CpT; N is any nucleotide; and N₁ + N₂ is from about 0-26 bases.

12. An oligonucleotide as claimed in claim 11, wherein N₁ and N₂ do not contain a CCGG quadmer or more than one CCG or CGG trimer; and the nucleic acid sequence is from about 8-30 bases in length.

13. An oligonucleotide as claimed in claim 11, wherein said oligonucleotide is 5'-
TCCATGACGTTCCTGACGTT-3' (SEQ ID NO: 3).

14. An oligonucleotide as claimed in claim 1, 2 or 3, wherein said method further comprises:
administering to the subject a therapeutically effective amount of the antigenic polypeptide.

15. A pharmaceutical composition comprising an immunostimulatory CpG oligonucleotide and a nucleic acid encoding an antigenic protein in a pharmaceutically acceptable carrier.


SEQUENCE LISTING

<110> Ottawa Hospital Research Institute et al

<120> Use of Nucleic Acids containing Unmethylated CpG
      Dinucleotide as an Adjuvant

<130> TSJ/34450EP2

<141> 1998-03-10

<150> US 60/040,376
<151> 1997-03-10

<160> 16

<170> PatentIn Ver. 2.1

<210> 1
<400> 1
000

<210> 2
<400> 2
000

<210> 3
<211> 20
<212> DNA
<213> Artificial Sequence

<220>
<223> Description of Artificial Sequence: misc_feature

<400> 3
tccatgacgt tcctgacgtt                                                20

<210> 4
<211> 20
<212> DNA
<213> Artificial Sequence

<220>
<223> Description of Artificial Sequence: misc_feature

<400> 4
tccatgtcgc tcctgatgct                                                20

<210> 5
<211> 20
<212> DNA
<213> Artificial Sequence

<220>
<223> Description of Artificial Sequence: misc_feature

<400> 5
tccatgtcgt tcctgatgct                                                20

<210> 6
<211> 24
<212> DNA
<213> Artificial Sequence

<220>
<223> Description of Artificial Sequence: misc_feature

<400> 6
tcgtcgtttt gtcgttttgt cgtt                                              24


<210> 7
<211> 20
<212> DNA
<213> Artificial Sequence

<220>
<223> Description of Artificial Sequence: misc_feature

<400> 7
tcgtcgttgt cgttgtcgtt                                                   20


<210> 8
<211> 22
<212> DNA
<213> Artificial Sequence

<220>
<223> Description of Artificial Sequence: misc_feature

<400> 8
tcgtcgttgt cgttttgtcg tt                                                22


<210> 9
<211> 21
<212> DNA
<213> Artificial Sequence

<220>
<223> Description of Artificial Sequence: misc_feature

<400> 9
gcgtgcgttg tcgttgtcgt t                                                 21


<210> 10
<211> 21
<212> DNA
<213> Artificial Sequence

<220>
<223> Description of Artificial Sequence: misc_feature

<400> 10
tgtcgtttgt cgtttgtcgt t                                                 21

<210> 11
<211> 19
<212> DNA
<213> Artificial Sequence

<220>
<223> Description of Artificial Sequence: misc_feature

<400> 11
tgtcgttgtc gttgtcgtt                                                    19


<210> 12
<211> 14
<212> DNA
<213> Artificial Sequence

<220>
<223> Description of Artificial Sequence: misc_feature

<400> 12
tcgtcgtcgt cgtt                                                         14


<210> 13
<211> 20
<212> DNA
<213> Artificial Sequence

<220>
<223> Description of Artificial Sequence: misc_feature

<400> 13
tcctgtcgtt ccttgtcgtt                                                   20


<210> 14
<211> 20
<212> DNA
<213> Artificial Sequence

<220>
<223> Description of Artificial Sequence: misc_feature

<400> 14
tcctgtcgtt ttttgtcgtt                                                   20


<210> 15
<211> 21
<212> DNA
<213> Artificial Sequence

<220>
<223> Description of Artificial Sequence: misc_feature

<400> 15
tcgtcgctgt ctgcccttct t                                                 21

<210> 16
<211> 21
<212> DNA
<213> Artificial Sequence

<220>
<223> Description of Artificial Sequence: misc_feature

<400> 16
tcgtcgctgt tgtcgtttct t                                                21